**Europäisches Patentamt**

**European Patent Office**

**Office européen des brevets**

(19)

(11) Veröffentlichungsnummer: **0 100 769**
**A1**

(12) **EUROPÄISCHE PATENTANMELDUNG**

(21) Anmeldenummer: **82107111.5**

(22) Anmeldetag: **06.08.82**

(51) Int. Cl.³: **A 61 M 11/00**
**//B05B17/06, F24F6/12**

(43) Veröffentlichungstag der Anmeldung:
**22.02.84 Patentblatt 84/8**

(84) Benannte Vertragsstaaten:
**AT BE CH DE FR GB IT LI LU NL SE**

(71) Anmelder: **Klarhorst, Günter**
**Eisgrundstrasse 7**
**D-4800 Bielefeld 18(DE)**

(71) Anmelder: **Vaillant, Michael**
**Kleppingstrasse 9-11**
**D-4600 Dortmund 1(DE)**

(72) Erfinder: **Klarhorst, Günter**
**Eisgrundstrasse 7**
**D-4800 Bielefeld 18(DE)**

(74) Vertreter: **Schirmer, Siegfried**
**Osningstrasse 10**
**D-4800 Bielefeld 1(DE)**

(54) **Vorrichtung zum Erzeugen eines Aerosols.**

(57) Bei der Vorrichtung sind das Lüfterrad (4) und das Bakterienfilter (5), die jeweils in einem Autoklaven oder einem Sterilisator steril gemacht werden müssen, zu einer Baueinheit zusammengeschlossen und über eine Steckverbindung neben der Vernebelungskammer (1) abnehmbar auf der Außenseite des Gehäuses (14) angeordnet.

In der Vernebelungskammer (1) sind zwei senkrecht verlaufende Luftkanäle (6) angeordnet, über die die Luft dem oberen Ende der Vernebelungskammer (1) zugeführt wird. Eine Verteilung der Luft erfolgt über einen mit Öffnungen (34) versehenen Verteilerring (35). Am Kammerdeckel (7) sind Austrittsöffnungen (37) und oberhalb dieser Austrittsöffnungen (37) eine mit Lochungen (38) versehene Ring-Kragscheibe (39) angeordnet.

Patentanwalt
**Dipl.-Ing. Siegfried Schirmer**

Taubenstraße 11a
4800 Bielefeld 1
Telefon (0521) 29 57 62

0100769

den 28.7.1982
246/28-3  S/b

Anmelder:

Günter Klarhorst
Eisgrundstraße 7

4800 Bielefeld 18

Michael Vaillant
Kleppingstraße 9-11

4600 Dortmund 1

Vorrichtung zum Erzeugen eines Aerosols

Die Erfindung betrifft eine Vorrichtung zum Erzeugen eines Aerosols mit einer eine Flüssigkeit mittels eines Schwingungserzeugers zerstäubenden und das Aerosol abgebenden Vernebelungskammer und einem von einem Motor angetriebenen, in einer gesonderten Kammer untergebrachten Lüfterrad, welches Luft über ein Bakterienfilter ansaugt und die angesaugte Luft über eine Verbindungsleitung dem oberen Teil der Vernebelungskammer zuführt, wobei das Lüfterrad über eine durch eine zwischen Motor und Lüfterrad angeordnete Trennwand hindurchwirkende Magnetkupplung mit dem Motor verbunden ist.

Ein Aerosol ist ein Gas, insbesondere Luft, das feste oder flüssige Schwebstoffe in feinstverteilter Form enthält. Aerosole aus Wasser und wäßrigen Lösungen mit Tröpfchengrößen von 1 bis 5 $\mu$m werden häufig in der Medizin verwendet, wobei den wäßrigen Lösungen zur Herstellung des Aerosols wasserlösliche Medikamente zugegeben werden können. Die in der Medizin verwendeten Aerosole aus Wasser und wäßrigen Lösungen dienen unter anderem zur Erhöhung der Luftfeuchtigkeit in Operationsräumen und Intensivstationen und zur Befeuchtung des Atmungstraktes von Patienten. Es ist hierbei wesentlich, daß die Aerosole keimfrei sind, um eventuelle Infektionen

- 2 -

zu vermeiden. Keimfreie Aerosole lassen sich jedoch nur mit Vorrichtungen erzielen, die selbst keimfrei arbeiten.

Eine Vorrichtung der aufgezeigten Gattung ist aus der DE-AS 28 43 756 bekannt. Bei dieser Vorrichtung ist das Lüfterrad in einer auswechselbaren Kassette untergebracht, die in einem Führungsrahmen des Gehäuses verschiebbar geführt und arretierbar ist. Mit Hilfe von Blattfedern und Einbuchtungen ist diese Kassette in ihrer Funktionsstellung festlegbar. Der Eintrittsöffnung der Lüfterradkammer ist ein Bakterienfilter vorgelagert, das zur Vermeidung des Ansaugens von Luft unter Umgehung des Bakterienfilters dicht angeschlossen sein muß.

Das in der Ansaugöffnung zum Lüfterrad angeordnete Bakterienfilter kann nicht verhindern, daß sich im Lüfterrad und im umgebenden Raum Krankheitserreger ansammeln, die auf dem Weg über die Vernebelungskammer in das Aerosol gelangen und eine Infektionsgefahr darstellen. Diese Gefahr ist vor allem dann gegeben, wenn das Bakterienfilter nicht dicht angeschlossen ist. Eine öftere Autoklavierung der Lüfterkassette und des Bakterienfilters ist erforderlich. Hierzu muß zunächst das Bakterienfilter seitlich aus dem Gehäuse und anschließend die Kassette nach oben aus dem Gehäuse herausgezogen werden.

Damit die gesamte Vorrichtung stets voll funktionsfähig ist, müssen drei Einzelteile, nämlich die Vernebelungskammer, die Lüfterradkassette und das Bakterienfilter jeweils entfernt und in einem Autoklaven oder einem Sterilisator steril gemacht werden. Diese Arbeit ist zeitaufwendig und erfordert größte Sorgfalt. Eine Gewähr dafür, daß alle drei Einzelteile stets einwandfrei steril gemacht und voll funktionsfähig wieder eingebaut worden sind, gibt es nicht. Es bleibt überwiegend der Sorgfalt des entsprechenden Personals überlassen, daß alle Teile steril und ordnungsgemäß wieder eingebaut werden.

Die Luftzuführung von der Lüfterradkammer zur Vernebelungskammer erfolgt über einen Verbindungsschlauch, der außerhalb der gesamten Vorrichtung verläuft und am oberen Ende der Vernebelungskammer angeschlossen ist. Eine gleichmäßige Verteilung der nur über eine Verbindungsleitung zugeführten Luft in der Vernebelungskammer ist nicht gewährleistet.

Der Erfindung liegt die Aufgabe zugrunde, eine Vorrichtung der aufgezeigten Gattung so auszubilden, daß der Aus- und Einbau der Elemente zum Zwecke der Zuführung zum Autoklaven oder Sterilisator erleichtert und vereinfacht ist und auf einen außerhalb der Vorrichtung verlaufenden Luft-Verbindungsschlauch verzichtet werden kann bei gleichzeitiger Erreichung einer gleichmäßigen Luftverteilung in der Vernebelungskammer.

Diese Aufgabe wird erfindungsgemäß dadurch gelöst, daß das in der gesonderten Kammer untergebrachte Lüfterrad und das Bakterienfilter zu einer Baueinheit zusammengeschlossen und auf der Außenseite des Gehäuses neben der Vernebelungskammer abnehmbar angeordnet sind, wobei die zu einer Baueinheit zusammengeschlossenen Teile und die Vernebelungskammer jeweils über eine Steckverbindung an das Gehäuse anschließbar sind und in der Vernebelungskammer ein oder mehrere senkrecht verlaufende Luftkanäle angeordnet sind, die unten mit der Lüfterradkammer in Verbindung stehen und oben an einem Halterungsring angeschlossen sind. In Ausgestaltung der Erfindung ist auf dem Halterungsring ein mit Öffnungen versehener Luft-Verteiler- und Aufnahmering für den Kammerdeckel angeordnet. Bei einer vorteilhaften Ausführungsform der Erfindung weist der Kammerdeckel im Abstand von seiner oberen Begrenzung Austrittsöffnungen auf, wobei oberhalb dieser Austrittsöffnungen eine mit Lochungen versehene Ring-Kragscheibe angeordnet sein kann. Weitere vorteilhafte Ausgestaltungen der Erfindung sind in den übrigen Unteransprüchen gekennzeichnet.

Durch die erfindungsgemäße Ausbildung ist die Zuführung der zu reinigenden Elemente zum Autoklaven oder Sterilisator vereinfacht. Es müssen jeweils nur die Vernebelungskammer und die zu einer Einheit zusammengeschlossenen Teile Lüfterrad und Bakterienfilter aus der auf der Oberfläche des Gehäuses vorgesehenen Steckverbindung gelöst werden. Durch den Einbau ist die Gewähr einer einwandfreien Funktion gegeben. Die Luftzuführung innerhalb der Vernebelungskammer ist gegen äußere Einwirkungen geschützt. Gleichzeitig wird erfindungsgemäß eine gleichmäßige Luftverteilung am oberen Ende der Vernebelungskammer erreicht.

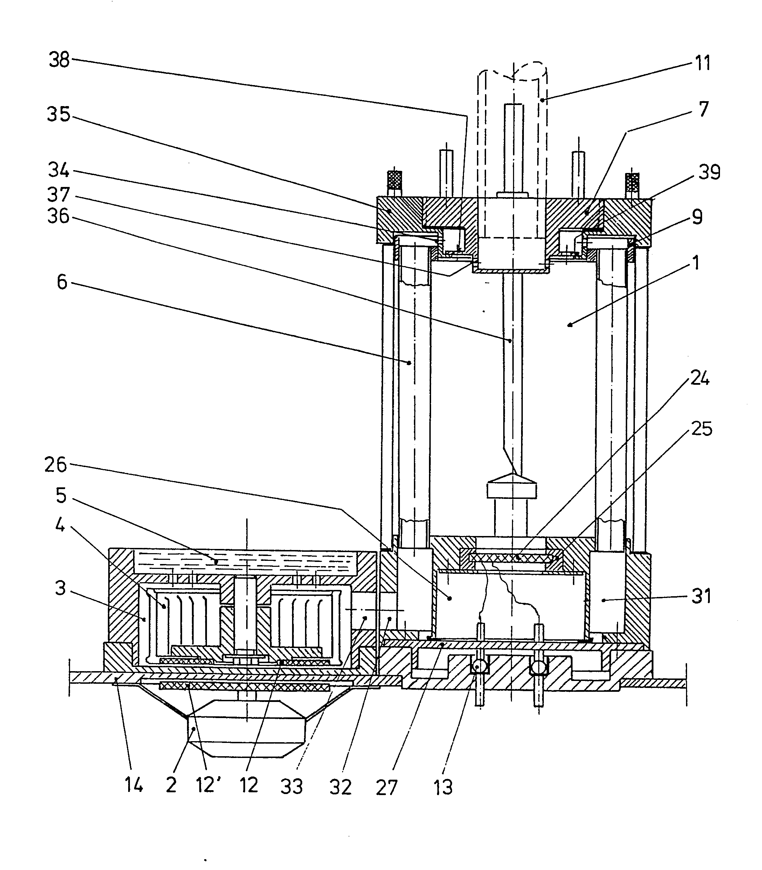

Ein Ausführungsbeispiel der Erfindung ist in der Zeichnung dargestellt und wird im folgenden näher beschrieben. Die einzige Figur zeigt einen schematischen Vertikalschnitt durch die wesentlichsten Teile der Vorrichtung.

Auf dem Gehäuse 14 der Vorrichtung ist das in der Lüfterradkammer 3 befindliche Lüfterrad 4 mit dem Bakterienfilter 5, die beide zu einer Baueinheit zusammengeschlossen sind, mittels einer Steckverbindung neben der Vernebelungskammer 1 abnehmbar angeordnet. Die Vernebelungskammer 1 ist ebenfalls über eine Steckverbindung 13 an das Gehäuse 14 angeschlossen.

Die Zerstäubung der Flüssigkeit erfolgt in der Vernebelungskammer 1 mit Hilfe des Ultraschall-Zerstäubereffektes, wobei die Flüssigkeit zu longitudinalen Schwingungen angeregt wird. Als Ultraschallschwingungserreger dient eine Quarzscheibe 24 mit Haltering 25, die an der unteren Begrenzung der Vernebelungskammer 1 in einer Schwingungskammer 26 angeordnet ist. Am Boden 27 der Schwingungskammer 26 ist die Steckverbindung 13 angeordnet. Die Schwingungskammer 26 ist von einem Luftkanal 31 umgeben, der eine Öffnung 32 zur Zuführung der angesaugten Luft aufweist. Diese Zuführöffnung 32 ist deckungsgleich mit der Austrittsöffnung 33 der Lüfterradkammer 3.

Die zu zerstäubende Flüssigkeit kann Wasser oder eine wäßrige Lösung mit wasserlöslichen Medikamenten sein. An die Vernebelungskammer 1 ist ein Abgabeschlauch 11 angeschlossen, aus welchem das Aerosol austritt und gegebenfalls dem Atmungsvorgang eines Patienten zugeführt wird. Mit oder auch ohne Abgabeschlauch 11 kann das Aerosol auch der Umgebungsluft zugeführt werden. Die Verwendung der erfindungsgemäßen Vorrichtung ist nicht auf den medizinischen Sektor begrenzt. Der Einsatz kann auch da erfolgen, wo eine Erhöhung der Luft-

feuchtigkeit erwünscht oder erforderlich ist, z.B. in der Nahrungsmittelindustrie, bei der Lederverarbeitung, in Museen, Bibliotheken und EDV-Räumen. Außerdem lassen sich in gewissen Grenzen auch optische Wirkungen erzielen.

Der Motor 2, der durch eine beliebige Energiequelle antreibbar ist, z.B. durch eine Batterie, ist von der Lüfterradkammer 3 luft- und wasserdicht getrennt. Diese Trennung erfolgt durch das durchgehende Gehäuse 14, das an der entsprechenden Stelle eine Stärkereduzierung aufweist. Es ist auch möglich, zur luft- und wasserdichten Trennung eine gesonderte Sicherheitsfolie zu verwenden.

Die Verbindung zwischen Motor 2 und Lüfterrad 4 erfolgt über eine Magnetkupplung. Diese Magnetkupplung besteht aus den beiden Magnetscheiben 12 und 12'. Das Lüfterrad 4 ist ein Radiallüfter, welcher die Luft axial ansaugt und radial abgibt.

In der Vernebelungskammer 1 sind zwei senkrecht verlaufende Luftkanäle 6 angeordnet, die unten mit der Lüfterradkammer 3 in Verbindung stehen und oben an einem Halterungsring 9 angeschlossen sind. Auf diesem Halterungsring 9 ist ein mit Öffnungen 34 versehener Verteiler- und Aufnahmering 35 für den Kammerdeckel 7 lösbar angeschlossen. Am Verteiler- und Aufnahmering 35 ist ein Niveaustandsröhrchen 36 angeordnet. Am Kammerdeckel 7 sind Austrittsöffnungen 37 vorgesehen. Der Kammerdeckel 7 besitzt gleichzeitig einen Anschluß für den Abgabeschlauch 11. Die Austrittsöffnungen 37

0100769

des Kammerdeckels 7 sind im Abstand von der oberen Begrenzung angeordnet, wobei oberhalb der Austrittsöffnungen 37 eine mit Lochungen 38 versehene Ring-Kragscheibe 39 vorgesehen ist.

- Patentansprüche -
- 8 -

Patentanwalt
Dipl.-Ing. Siegfried Schirmer

Taubenstraße 12a
4800 Bielefeld 1  **0100769**
Telefon (0521) 295782

- 8 -

den 28.7.1982
246/28-3  S/b

Anmelder:

Günter Klarhorst
Eisgrundstraße 7
4800 Bielefeld 18

Michael Vaillant
Kleppingstraße 9-11
4600 Dortmund 1

Patentansprüche:

1. Vorrichtung zum Erzeugen eines Aerosols mit einer eine Flüssigkeit mittels eines Schwingungserzeugers (24;25) zerstäubenden und das Aerosol abgebenden Vernebelungskammer (1) und einem von einem Motor (2) angetriebenen, in einer gesonderten Kammer (3) untergebrachten Lüfterrad (4), welches Luft über ein Bakterienfilter (5) ansaugt und die angesaugte Luft über eine Verbindungsleitung dem oberen Teil der Vernebelungskammer (1) zuführt, wobei das Lüfterrad (4) über eine durch eine zwischen Motor (2) und Lüfterrad (4) angeordnete Trennwand hindurchwirkende Magnetkupplung (12;12') mit dem Motor (2) verbunden ist, dadurch gekennzeichnet, daß das Lüfterrad (4) und das Bakterienfilter (5) zu einer Baueinheit zusammengeschlossen und auf der Außenseite des Gehäuses (14) neben der Vernebelungskammer (1) abnehmbar angeordnet sind.

2. Vorrichtung nach Anspruch 1, dadurch gekennzeichnet, daß die zu einer Baueinheit zusammengeschlossenen Teile (4;5) und die Vernebelungskammer (1) jeweils über eine Steckverbindung (13) an das Gehäuse (14) anschließbar sind.

3. Vorrichtung nach Anspruch 1 oder 2, dadurch gekennzeichnet, daß in der Vernebelungskammer (1) ein oder mehrere senkrecht verlaufende Luftkanäle (6) angeordnet sind, die unten mit der Lüfterradkammer (3) in Verbindung stehen und oben an einem Halterungsring (9) angeschlossen sind.

4. Vorrichtung nach einem der Ansprüche 1 bis 3, dadurch gekennzeichnet, daß unterhalb der Vernebelungskammer (1) der an sich bekannte Schwingungserzeuger (24;25) in einer besonderen Schwingungskammer (26) angeordnet ist, auf dessen Boden (27) die Steckverbindung (13) angeordnet ist.

5. Vorrichtung nach einem der Ansprüche 1 bis 4, dadurch gekennzeichnet, daß die Schwingungskammer (26) von einem Luftkanal (31) umgeben ist, der eine Öffnung (32) zur Zuführung der angesaugten Luft aufweist.

6. Vorrichtung nach einem der Ansprüche 1 bis 5, dadurch gekennzeichnet, daß die Zuführöffnung (32) des Luftkanals (31) deckungsgleich mit der Austrittsöffnung (33) der Lüfterradkammer (3) angeordnet ist.

7. Vorrichtung nach einem der Ansprüche 1 bis 6, dadurch gekennzeichnet, daß auf dem Halterungsring (9) ein mit Öffnungen (34) versehener Verteiler- und Aufnahmering (35) für den Kammerdeckel (7) angeordnet ist.

8. Vorrichtung nach Anspruch 7, dadurch gekennzeichnet, daß der Verteiler- und Aufnahmering (35) lösbar am Halterungsring (9) angeschlossen ist.

9. Vorrichtung nach einem der Ansprüche 1 bis 8, dadurch gekennzeichnet, daß am Verteiler- und Aufnahmering (35) ein Niveaustandsröhrchen (36) angeordnet ist.

10. Vorrichtung nach einem der Ansprüche 1 bis 9, dadurch gekennzeichnet, daß der Kammerdeckel (7) Austrittsöffnungen (37) und einen Anschluß für einen Abgabeschlauch (11) aufweist.

11. Vorrichtung nach Anspruch 10, dadurch gekennzeichnet, daß die Austrittsöffnungen (37) des Kammerdeckels (7) im Abstand von seiner oberen Begrenzung angeordnet sind.

12. Vorrichtung nach Anspruch 10 und 11, dadurch gekennzeichnet, daß oberhalb der Austrittsöffnungen (37) des Kammerdeckels (7) eine mit Lochungen (38) versehene Ring-Kragscheibe (39) angeordnet ist.

13. Vorrichtung nach einem der Ansprüche 1 bis 12, dadurch gekennzeichnet, daß das Lüfterrad (4) ein Radiallüfter ist.

- Zusammenfassung -

Europäisches Patentamt

**EUROPÄISCHER RECHERCHENBERICHT**

**0100769**
Nummer der Anmeldung

EP 82 10 7111

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | A 61 M 11/00 // |
| X | DE-A-2 941 554 (H. HENSE) * Figuren; Seite 3, Zeile 1 - Seite 4, Zeile 7; Seite 6, Zeilen 8-16; Seite 6, Zeile 23 - Seite 7, Ende * | 1,13 | B 05 B 17/06 F 24 F 6/12 |
| | --- | | |
| A | DE-C- 98 263 (McGREGOR MED. CY. INC.) * Figur 1; Seite 1, linke Spalte, Absatz 3 - rechte Spalte, Absatz 1 * | 3 | |
| | --- | | |
| A | DE-A-3 043 325 (I. BRUGGER) * Text und Figuren * | 1,2 | |
| | ----- | | |

| RECHERCHIERTE SACHGEBIETE (Int. Cl. ³) |
|---|
| A 61 M B 05 B F 24 F |

Der vorliegende Recherchenbericht wurde für alle Patentansprüche erstellt.

| Recherchenort | Abschlußdatum der Recherche | Prüfer |
|---|---|---|
| DEN HAAG | 24-01-1983 | VEREECKE A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument. das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82